# EUROPÄISCHE PATENTSCHRIFT

(11) **EP 4 017 467 B1**
(45) Veröffentlichungstag und Bekanntmachung des Hinweises auf die Patenterteilung: **20.05.2026**
(21) Anmeldenummer: 20761764.8
(22) Anmeldetag: 18.08.2020
(51) Int. Cl.: A61K 8/73, A61K 8/06, A61Q 19/00

(54) **KOSMETISCHE O/W-EMULSION MIT SUCCINOGLUCAN**
COSMETIC O/W EMULSION CONTAINING SUCCINOGLUCAN
ÉMULSION EW COSMÉTIQUE CONTENANT DU SUCCINOGLUCANE

(30) Priorität: 19.08.2019 DE 102019212327
(43) Veröffentlichungstag der Anmeldung: 29.06.2022
(73) Patentinhaber: Beiersdorf AG, 22529 Hamburg (DE)
(72) Erfinder: PIRADASHVILI, Keti, 22111 Hamburg (DE); RASCHKE, Thomas, 25421 Pinneberg (DE); PESCHKE, Lisa, 22303 Hamburg (DE)
(86) Internationale Anmeldenummer: PCT/EP2020/073038
(87) Internationale Veröffentlichungsnummer: WO 2021/032700

(56) Entgegenhaltungen:
- EP-A1- 1 576 943
- EP-A1- 2 308 459
- EP-A1- 3 329 903
- EP-A2- 2 630 947
- WO-A1-2013/061712
- DE-A1- 102011 077 060
- US-A1- 2014 030 298

## Beschreibung

Die vorliegende Erfindung betrifft eine kosmetische Zubereitung enthaltend Succinoglucan.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren haben dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z. B. das Entstehen von Fältchen, Falten) verhindern und vermindern sollen.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um Emulsionen über einen längeren Zeitraum stabil zu halten und eine Entmischung der Phasen zu verhindern, werden den Emulsionen sogenannte Emulgatoren zugesetzt. Bei Emulgatoren handelt es sich in der Regel um Moleküle mit einem polaren, hydrophilen Strukturelement und einem unpolaren, lipophilen Strukturelement. Ende der vierziger Jahre wurde ein System entwickelt, das die Auswahl von Emulgatoren erleichtern sollte. Jedem Emulgator wird ein so genannter HLB-Wert (eine dimensionslose Zahl zwischen 0 und 20) zugeschrieben, der angibt, ob eine bevorzugte Wasser- oder Öllöslichkeit vorliegt. Zahlen unter 8 kennzeichnen öllösliche, hydrophobe Emulgatoren, Zahlen über 8 wasserlösliche, hydrophile.

Bei der Herstellung einer Emulsion wird die Wasserphase mit der Lipidphase (Ölphase) unter Rühren vereinigt, wobei die Tröpfchen der inneren Phase der Emulsion auf eine Größe von unter 10 µm zerkleinert werden müssen, damit die Emulsion stabil wird.

Bei der Herstellung von O/W-Emulsionen mit hydrophilen Emulgatoren (Emulgatoren mit einem HLB-Wert von größer/gleich 8) tritt häufig das Problem auf, dass bei der Verwendung von überwiegend mittelpolaren bis polaren Lipiden in der Ölphase (d. h. Lipide mit einer Grenzflächenspannung gegen Wasser von kleiner als 30 mN/m) eine unerwünschte Phasenumkehr stattfinden kann. Ferner neigen derartige O/W-Emulsionen bei der Lagerung zur Phasenumkehr, d. h. die Zubereitungen sind häufig nicht besonders lagerstabil.

Es war daher die Aufgabe der vorliegenden Erfindung, eine O/W-Emulsion mit hydrophilen Emulgatoren bzw. ein Herstellungsverfahren für eine O/W-Emulsion mit hydrophilen Emulgatoren zu entwickeln, deren Neigung zur Phasenumkehr während der Herstellung und/oder Lagerung deutlich reduziert ist.

Die überraschende Lösung dieser Aufgabe findet sich in Anspruch 1.

Zwar kennt der Stand der Technik die EP 1576943 A1, EP 2308459 A1, WO 2013/061712 A1, US 2014/030298 A1, DE 102011077060 A1 und EP 3329903 A1, doch konnten diese Schriften nicht den Weg zur vorliegenden Erfindung weisen.

Succinoglucan ist ein mit Bernsteinsäure teilweise verestertes Polysaccharid, welches beispielsweise als Fermentationsproduct von Agrobacterium tumefaciens gewonnen werden kann. Succinoglucan wird dabei) als mikrobielles Exopolysaccharid in das umgebende Medium abgegeben.

Das Rückgrat des Succinoglucans bilden dabei vorwiegend Glucosemonomere, vorteilhaft kann allerdings auch Galactose einpolymerisiert sein. Ein besonders vorteilhaftes Succinoglucan weist molare Verhältnisse von Glucose zu Galactose wie 8 - 6 : 1, insbesondere ungefähr wie 7 : 1 auf.

Erfindungsgemäß vorteilhaft ist im statistischen Mittel jedes achte bis zwölfte Zuckermolekül des polymeren Rückgrates mit einem Molekül Bernsteinsäure verestert, bevorzugt etwa jedes zehnte Zuckermolekül.

Es kann auch vorteilhaft sein, zuzüglich weitere funktionelle Moleküle an die Zuckermoleküle zu binden, beispielsweise Brenztraubensäure, die über eine Acetalbildung angekoppelt werden kann.

Erfindungsgemäß vorteilhaft ist im statistischen Mittel jedes fünfte bis zwanzigste Zuckermolekül des polymeren Rückgrates an ein Molekül Brenztraubensäure gekoppelt, bevorzugt etwa jedes achte Zuckermolekül.

Die chemische Struktur des Succinoglucans wird wie folgt veranschaulicht:

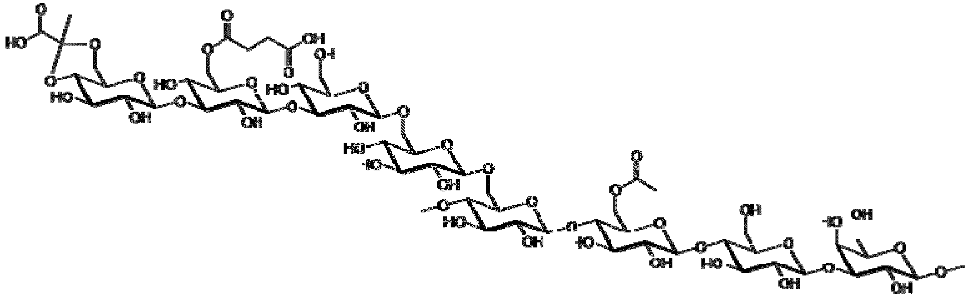

Succinoglucan ist in Wasser gut löslich und kann als Verdicker eingesetzt werden. Der Einsatz von Succinoglucan in kosmetischen Emulsionen ist dem Fachmann an sich bekannt, doch konnte der Stand der Technik nicht den Weg zur vorliegenden Erfindung weisen.

Es ist erfindungsgemäß vorteilhaft, wenn die erfindungsgemäße Emulsion dadurch gekennzeichnet ist, dass der Gehalt an Succinoglucan in der Emulsion von 0,001 bis 5,0 Gew.-% und bevorzugt zwischen 0,01 und 1,5 Gew.-%, besonders bevorzugt zwischen 0,1 und 1,0 Gew.-%, jeweils bezogen auf das Gesamtgewicht der Emulsion, beträgt.

Es ist erfindungsgemäß vorteilhaft, wenn die Emulsion einen oder mehrere Öl-in-Wasser-Emulgatoren (O/W-Emulgatoren) in einer Gesamtkonzentration von 0,05 bis 5,0 Gew.-% und bevorzugt in einer Gesamtkonzentration von 2,0 bis 4,0 Gew.-%, jeweils bezogen auf das Gesamtgewicht der Emulsion, beträgt.

Es ist erfindungsgemäß besonders vorteilhaft, wenn die erfindungsgemäße Emulsion dadurch gekennzeichnet ist, dass der Gehalt an einem oder mehreren anionischen Emulgatoren in der Emulsion von 0,001 bis 5,0 Gew.-% und bevorzugt zwischen 0,05 und 2 Gew.-%, besonders bevorzugt zwischen 0,1 und 1,0 Gew.-%, jeweils bezogen auf das Gesamtgewicht der Emulsion, beträgt.

Es ist erfindungsgemäß besonders vorteilhaft, wenn die erfindungsgemäße Emulsion dadurch gekennzeichnet ist, dass der Gehalt an einem oder mehreren nichtionischen Emulgatoren in der Emulsion von 0,001 bis 5,0 Gew.-% und bevorzugt zwischen 0,05 und 3 Gew.-%, besonders bevorzugt zwischen 0,1 und 2 Gew.-%, jeweils bezogen auf das Gesamtgewicht der Emulsion, beträgt.

Es ist erfindungsgemäß, wenn die erfindungsgemäße Emulsion dadurch gekennzeichnet ist, dass als O/W-Emulgatoren Glycerylstearatcitrat (CAS 39175-72-9, INCI Glyceryl Stearate Citrate, z. B. Imwitor 370 der Firma Hüls), Polyglyceryl-3 Methylglucosedistearat (INCI Polyglyceryl Methyl Glucose Distearate, z. B. Tego Care 450 der Firma Evonik), Cetearylglucoside und/oder Polyethylenglycol(2000)monostearat (INCI; PEG-40 stearate) eingesetzt werden.

In einer weiteren erfindungsgemäßen Ausführungsform enthält die erfindungsgemäße Emulsion Kalium Cetylphosphat, Natrium Cetearylsulfat, Natrium Stearylglutamat, Alkalisalze von gesättigten Fettsäuren der Kettenlänge C14-C22, insbesondere Natrium Stearat als anionischen Emulgator.

In einer weiteren erfindungsgemäßen Ausführungsform enthält die erfindungsgemäße Emulsion einen oder mehrere Polyglycerylfettsäureester als nichtionische Emulgatoren. Der erfindungsgemäß besonders bevorzugte Polyglycerylfettsäureester ist dabei Polyglyceryl-10 Stearat (INCI Polyglyceryl-10 Stearate).

Es ist erfindungsgemäß besonders vorteilhaft, wenn die erfindungsgemäße Emulsion dadurch gekennzeichnet ist, dass der Gehalt an ein oder mehreren anionischen Emulgatoren in der Emulsion von 0,001 bis 5,0 Gew.-% und bevorzugt zwischen 0,05 und 2 Gew.-%, besonders bevorzugt zwischen 0,1 und 1,0 Gew.-%, jeweils bezogen auf das Gesamtgewicht der Emulsion, beträgt.

Es ist erfindungsgemäß besonders vorteilhaft, wenn die erfindungsgemäße Emulsion dadurch gekennzeichnet ist, dass der Gehalt an ein oder mehreren nichtionischen Emulgatoren, insbesondere einen oder mehrere Polyglycerylfettsäureester, besonders Polyglyceryl-10 Stearat in der Emulsion von 0,001 bis 5,0 Gew.-% und bevorzugt zwischen 0,05 und 3 Gew.-%, besonders bevorzugt zwischen 0,1 und 2,0 Gew.-%, jeweils bezogen auf das Gesamtgewicht der Emulsion, beträgt.

Die erfindungsgemäße Emulsion kann die Konsistenz einer Salbe, Creme oder dünnflüssigen Lotion aufweisen bzw. eine Salbe, Creme oder dünnflüssige Lotion darstellen.

Es ist erfindungsgemäß bevorzugt, wenn das erfindungsgemäße Verfahren in Form des Hot-Hot-Verfahrens durchgeführt wird. Bei diesem Verfahren werden eine heiße Öl- und Wasserphase miteinander vereinigt.

Dabei ist es erfindungsgemäß besonders bevorzugt, wenn das Verfahren bei einer Temperatur nicht über 85 °C durchgeführt wird.

Die Emulsion kann erfindungsgemäß vorteilhaft als Salbe, Creme oder Lotion (ggf. sprühbar) vorliegen.

Die Emulsion kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Emulsion, erfindungsgemäß.

Die Wasserphase der erfindungsgemäßen Emulsion kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Hexandiol, Octandiol, Glycerylcaprylat, Ethylhexylglycerin, Methylpropandiol, Propylenglykolmonomethyl, -monoethyl- oder -monobutylether, Diethylenglykolmonomethyl- oder monoethylether, und analoge Produkte, außerdem Phenoxyethanol, Hydroxyacetophenone, Benzylalkohol, Benzethonium chlorid, Polymere, Schaumstabilisatoren, Elektrolyte, Selbstbräuner, Repellentien sowie insbesondere ein oder mehrere Verdickungsmittel, welches oder welche vorteilhaft gewählt werden können aus der Gruppe der Polysaccharide bzw. deren Derivate, z.B. Xanthangummi, Carrageenan, Gellangummi, Stärken und Cellulosen, Hyaluronsäure.

Die erfindungsgemäße Ölphase kann alle herkömmlichen Bestandteile von in der Kosmetik eingesetzten Öl-, Fett- und Wachskomponenten enthalten.

Die erfindungsgemäße Emulsion kann erfindungsgemäß vorteilhaft kosmetische Wirk- und Pflegestoffe enthalten, beispielsweise die nach der Kosmetikverordnung zugelassenen UV-Lichtschutzfilter. Derartige Wirkstoffe können erfindungsgemäß vorteilhaft in Konzentrationen von 0,01 bis 30 Gew.-%, bezogen auf das Gesamtgewicht der Zubereitung in dieser enthalten sein.

Als weitere Pflegestoffe können insbesondere Niacinamid, Panthenol, Aloe vera, Vitamin E, Tocopherylacetat, Vitamin A, Vitamin A-Derivate, Vitamin C, Vitamin C-Derivate, Coenzym Q10 (Ubiquinone), Kreatin, Taurin, Folsäure, Alpha-Glycosylrutin, Aminosäuren (z.B. Glycin, Histidin, Alanin, Arginin), Carnosin, ungesättigte Fettsäuren und deren Ester, insbesondere Di- und Triglyceride (z.B. γ-Linolensäure, Linolsäure, Ölsäure), Mannose, Magnolia Rindenextrakt, Magnolol, Honokiol, Extrakte der Mariendistel, Silymarin, Urea, 4-Butylresorcinol, N-((2,4-Dihydroxyphenyl)thiazol-2-yl)-isobutyramid, Inhaltstoffe von Süßholzextrakten wie zum Beispiel Glycyrrhetinsäure und Licochalcone A in Konzentrationen von 0,001 bis 5 Gew.-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gew.-%, bezogen auf das Gesamtgewicht der Zubereitung eingesetzt.

Die erfindungsgemäße Zubereitung kann Parfümstoffe in beliebiger Zusammensetzung und Menge enthalten.

Die nachfolgenden Beispiele sollen die vorliegende Erfindung verdeutlichen ohne sie einzuschränken. Die Angaben beziehen sich stets auf Gew.-%, sofern nicht andere Angaben gemacht werden.

### Rezepturbeispiele:

| **Phase** | **Inhaltsstoffe** | **Bsp. 1** | **Bsp. 2** | **Bsp. 3** | **Bsp. 4** |
|---|---|---|---|---|---|
| Phase A | Glycerin | 5 | 5 | 5 | 5 |
| | Aqua | ad 100 | ad 100 | ad 100 | ad 100 |
| | Sodium cetearylsulfate | 0.2 | 0.2 | 0.2 | 0.2 |
| Phase B | Succinoglycan gum | - | 0.3 | - | - |
| | Xanthan gum | - | - | 0.3 | - |
| | Carrageenan | - | - | - | 0.3 |
| Phase C | Cetearyl alcohol | 2 | 2 | 2 | 2 |
| | Hydrogenated coco-glycerides | 2 | 2 | 2 | 2 |
| | Dicaprylyl ether | 8 | 8 | 8 | 8 |
| Phase D | Phenoxyethanol | 0.8 | 0.8 | 0.8 | 0.8 |
| | Methylparabene | 0.3 | 0.3 | 0.3 | 0.3 |
| | Trisodium EDTA | 0.2 | 0.2 | 0.2 | 0.2 |

| **Lagerung** | | | | | |
|---|---|---|---|---|---|
| Bei Raumtemperatur | Nach 24h | stabil | stabil | stabil | stabil |
| | Tag 7 | stabil | stabil | stabil | stabil |
| bei 40°C | Nach 24h | stabil | stabil | stabil | stabil |
| | Tag 7 | Phasentrennung nach 4 Tagen | stabil | Phasentrennung | Phasentrennung nach 4 Tagen |
| bei 50°C | Nach 24h | stabil | stabil | stabil | stabil |
| 12 h Zyklus von - 10°C bis 40°C | Tag 6 | Phasentrennung | stabil | stabil | stabil |

| **Phase** | **Inhaltsstoffe** | **Bsp. 5** | **Bsp. 6** | **Bsp. 7** | |
|---|---|---|---|---|---|
| Phase A | Glycerin | 5 | 5 | 5 | |
| | Aqua | ad 100 | ad 100 | ad 100 | |
| Phase B | Succinoglycan gum | - | 0.3 | - | |
| | Carrageenan | - | - | 0.3 | |
| Phase C | Cetearyl alcohol | 2 | 2 | 2 | |
| | Hydrogenated coco-glycerides | 2 | 2 | 2 | |
| | Dicaprylyl ether | 8 | 8 | 8 | |
| | Polyglyceryl-10 stearate | 0.9 | 0.9 | 0.9 | |
| Phase D | Phenoxyethanol | 0.8 | 0.8 | 0.8 | |
| | Methylparabene | 0.3 | 0.3 | 0.3 | |
| | Trisodium EDTA | 0.2 | 0.2 | 0.2 | |

| **Lagerung** | | | | | |
|---|---|---|---|---|---|
| Bei Raumtemperatur | Tag 1 | stabil | stabil | stabil | |
| | Tag 7 | stabil | stabil | Phasentrennung | |
| bei 40°C | Tag 1 | stabil | stabil | stabil | |
| | Tag 7 | Phasentrennung | stabil | Phasentrennung nach 4 Stunden | |
| bei 50°C | Tag 1 | Phasentrennung nach 5 Stunden | stabil | Phasentrennung nach 5 Stunden | |
| | Tag 7 | - | Phasentrennung nach 4 Tagen | - | |
| 12 h Zyklus von - 10°C bis 40°C | Tag 6 | Phasentrennung nach 5 Stunden | stabil | Phasentrennung | |

| **Beispiel A** | **Gew.-%** |
|---|---|
| O/W-Creme | |
| Natrium Stearoylglutamat | 0,3 |
| Glycerinmonostearat | 3 |
| C₁₂₋₁₅ Alkylbenzoat | 2 |
| Dicaprylylether | 2 |
| Octyldodecanol | 2 |
| Caprylsäure/Caprinsäuretriglyceride | 1 |
| Jojobaöl | 0,5 |
| Tocopherylacetat | 0,3 |
| N-((2,4-Dihydroxyphenyl)thiazol-2-yl)-isobutyramid, | 0,1 |
| Phenoxyethanol | 0,7 |
| Succinoglucan gum | 0,3 |
| Octandiol | 0,3 |
| Benzethonium chloride | 0,05 |
| Hydroxyacetophenone | 0,2 |
| Glycerin | 7 |
| Füllstoffe/ Additive (Tocopherol, EDTA) | 0,1 |
| Parfüm | q.s. |
| Wasser | ad 100 |

| **Beispiel B** | **Gew.-%** |
|---|---|
| O/W-Tagescreme | |
| Natrium Cetylstearylsulfat | 0,2 |
| Sorbitanmonostearat | 2 |
| Cetearylalkohol | 1 |
| C₁₂₋₁₅ Alkylbenzoat | 3 |
| Caprylsäure/Caprinsäuretriglyceride | 2 |
| Dicaprylylcarbonat | 2 |
| Octyldodecanol | 1 |
| Arganöl | 0,2 |
| Octocrylene | 2 |
| Butylmethoxydibenzoylmethan (z.B. Parsol^{®} 1789) | 1 |
| Octylsalicylat | 2 |
| Tapiocastärke | 0,5 |
| Glycyrrhetinsäure | 0.1 |
| Creatin | 0,1 |
| Phenoxyethanol | 0,8 |
| Hexandiol | 1 |
| Succinoglucan gum | 0,2 |
| Glycerin | 7 |
| Additive (Xanthan Gum, TiO2) | 0,6 |
| Parfüm | q.s. |
| Wasser | ad 100 |

### pH 6

| **Beispiel C** | **Gew.-%** |
|---|---|
| O/W-Creme | |
| Stearinsäure | 2 |
| Sorbitanmonostearat | 1 |
| Cetearylalkohol | 2 |
| Isopropylpalmitate | 3 |
| Ethylhexylstearat | 1 |
| Hydrierte Kokosfettglyceride (Hydrogenated Coco Glycerides) | 1 |
| C₁₂₋₁₅ Alkylbenzoat | 2 |
| Dibutyladipate | 1 |
| Niacinamid | 0,1 |
| Coenzym Q10 (Ubiquinon) | 0,1 |
| Milchsäure Natriumsalz | 0,2 |
| Methylpropandiol | 2 |
| Phenoxyethanol | 0,5 |
| Benzylalkohol | 0,2 |
| Succinoglucan gum | 0,5 |
| Hyaluronsäure, Natriumsalz | 0,1 |
| NaOH_{aq} | q.s. |
| Glycerin | 8 |
| Additive (EDTA, Carrageenan, Gellan gum HA) | 0,3 |
| Parfüm | q.s. |
| Wasser | ad 100 |

### pH 6

| **Beispiel D** | **Gew.-%** |
|---|---|
| O/W Creme | |
| Natrium Cetylstearylsulfat | 0,3 |
| Sorbitanmonostearat | 2 |
| Cetylalkohol | 1 |
| C₁₂₋₁₅ Alkylbenzoat | 2 |
| Sheabutter | 0,5 |
| Caprylsäure/Caprinsäuretriglyceride | 3 |
| Dicaprylylcarbonat | 2 |
| Dicaprylylether | 1 |
| Ethylhexylcocoate | 1 |
| Isohexadecan | 1 |
| Citronensäure Natriumsalz | qs. |
| Phenoxyethanol | 0,8 |
| Dipropylenglycol | 1 |
| Benzylalkohol | 0,3 |
| Glycyrrhiza inflata Extrakt (Licochalcone A) | 0,1 |
| Ethylhexylglycerin | 0,4 |
| Succinoglucan gum | 0,2 |
| EDTA | 0,2 |
| Ethanol | 1 |
| Glycerin | 5 |
| Additive (EDTA, Locust bean gum, Sclerotium gum) | 0,6 |
| NaOH_{aq} | q.s. |
| Parfüm | q.s. |
| Wasser | ad 100 |

### pH 5,2

| **Beispiel E** | **Gew.-%** |
|---|---|
| O/W-Creme | |
| Kalium Cetylphosphat | 1 |
| Glycerylstearate | 2 |
| Cetearylalkohol | 1 |
| Caprylsäure/Caprinsäuretriglyceride | 2 |
| C₁₂₋₁₅ Alkylbenzoat | 2 |
| Sonnenblumenöl | 0,5 |
| Cocosglyceride | 2 |
| Octyldodecanol | 1 |
| Triisostearin | 1 |
| Cetearylisononanoate | 1 |
| p-Hydroxybenzoesäuremethylester (Paraben) | 0,2 |
| Succinoglucan gum | 0,4 |
| Octandiol | 0,2 |
| N-((2,4-Dihydroxyphenyl)thiazol-2-yl)-isobutyramid | 0,1 |
| Glycerin | 5 |
| Panthenol | 1 |
| Additive (Sclerotium Gum, EDTA) | 0,5 |
| Parfüm | q.s. |
| Wasser | ad 100 |

| **Beispiel F** | **Gew.-%** |
|---|---|
| O/W-Creme | |
| Glycerylstearatcitrat | 2 |
| Stearylalkohol | 2 |
| C₁₂₋₁₅ Alkylbenzoat | 2 |
| Dicaprylylether | 2 |
| Octyldodecanol | 2 |
| Caprylsäure/Caprinsäuretriglyceride | 1 |
| Jojobaöl | 0,5 |
| Tocopherylacetat | 0,3 |
| Natriumascorbylphosphat | 0,1 |
| Phenoxyethanol | 0,7 |
| Succinoglucan gum | 0,4 |
| Magnolia Rindenextrakt | 0,1 |
| Glycerylcaprylat | 0,3 |
| Benzethonium chloride | 0,05 |
| Pentylenglycol | 2 |
| Glycerin | 7 |
| Füllstoffe/ Additive (Hydroxypropylmethylcellulose) | 0,5 |
| Neutralisationsmittel (Citronensäure, NaOH_{aq}) (pH 6 eingestellt) | q.s. |
| Parfüm | q.s. |
| Wasser | ad 100 |

| **Beispiel G** | **Gew.-%** |
|---|---|
| O/W Creme | |
| Polyglyceryl-10 stearate | 1,5 |
| Sorbitanstearat | 2 |
| Stearylalkohol | 2 |
| C₁₂₋₁₅ Alkylbenzoat | 2 |
| Sheabutter | 0,5 |
| Caprylsäure/Caprinsäuretriglyceride | 3 |
| Dicaprylylcarbonat | 2 |
| Dicaprylylether | 1 |
| Homosalate | 2 |
| Octocrylene | 5 |
| Octylsalicylat | 2 |
| 2,4-Bis-{[4-(2-Ethyl-hexyloxy)-2-hydroxy]-phenyl}-6-(4-methoxyphenyl)-1,3,5-triazin (Tinosorb^{®} S) | 1 |
| Benzylalkohol | 0,2 |
| Folsäure | 0,1 |
| Ethylhexylglycerin | 0,4 |
| Succinoglucan gum | 0,5 |
| Retinylpalmitat | 0,1 |
| Ethanol | 3 |
| Glycerin | 5 |
| Additive (EDTA, Sclerotium gum) | 0,6 |
| Neutralisationsmittel (Citronensäure, NaOH_{aq}) | q.s. |
| Parfüm | q.s. |
| Wasser | ad 100 |

### pH 5,0-5,5

| **Beispiel H** | **Gew.-%** |
|---|---|
| O/W-Creme | |
| Polyglyceryl-3 Methylglucose Distearate | 1,5 |
| Sorbitanmonostearat | 2 |
| Cetearylalkohol | 2 |
| Caprylsäure/Caprinsäuretriglyceride | 3 |
| C₁₂₋₁₅ Alkylbenzoat | 2 |
| Cocosglyceride | 2 |
| Octyldodecanol | 2 |
| p-Hydroxybenzoesäuremethylester (Paraben) | 0,2 |
| Succinoglucan gum | 0,2 |
| Ethylhexylglycerin | 0,3 |
| Methylpropandiol | 3 |
| Glycerin | 5 |
| Panthenol | 1 |
| Glycerylglucose | 0,5 |
| Additive (Silica, Benzethoniumchlorid, o-Cymen-5-ol, Neutralisationsmittel) | 0,8 |
| Parfüm (enthaltend Citral, Carvone, Carvacrol, Carveol, Geraniol, Nerol, Thymol, Menthon, Zimtaldehyd, Vanillin, p-Anisaldehyd, Eugenol, Anisylalkohol, Isopropylmethylphenol, Terpineol, Heliortopin) | q.s. |
| Wasser | ad 100 |

### pH 5-6

| **Beispiel** I Getönte Tagescreme | Gew.-% |
|---|---|
| Polyglyceryl-3 Methylglucose Distearate | 2 |
| Glyceryl Stearate | 1 |
| Cetylalkohol | 2 |
| Octyldodecanol | 4 |
| Cetearyl Isononanoate | 4 |
| Dicaprylyl Ether | 3 |
| Phenoxyethanol | 0,5 |
| Octandiol | 0,2 |
| Vitamin E Acetat | 0,2 |
| Succinoglucan gum | 0,3 |
| Glycerin | 8 |
| Neutralisationsmittel (Citronensäure und / oder NaOH_{aq}) (pH 6 eingestellt) | q.s. |
| Silica beschichtete anorganischen Pigmente [(CI 77499, CI 77491, CI 77891, CI 77492) + Silica] - PRIMROSE | 6 |
| Wasser | Ad 100 |

## Patentansprüche

1. Kosmetische Öl-in-Wasser-Emulsionen (O/W-Emulsionen) enthaltend neben einer Wasserphase eine oder mehrere darin dispergierte Ölphasen sowie
a) einen oder mehrere anionische Emulgatoren und/oder
b) einen oder mehrere nichtionische Emulgatoren und
c) einem oder mehreren Succinoglucanen,
wobei als O/W-Emulgatoren Polyglyceryl-3 Methylglucosedistearat (INCI Polyglyceryl Methyl Glucose Distearate, Kalium Cetylphosphate, Natrium Cetearylsulfat, Cetearylglucoside, Natrium Stearylglutamat, Kalium Cetylphosphate, Alkalisalze von gesättigten Fettsäuren der Kettenlänge C14-C22 oder ein oder mehrere Polyglycerylfettsäureester eingesetzt werden.

2. O/W-Emulsionen nach Anspruch 1, **dadurch gekennzeichnet, dass** der Gehalt an Succinoglucan in der Emulsion von 0,01 bis 1,0 Gew.-%, bezogen auf das Gesamtgewicht der Emulsion, beträgt.

3. O/W-Emulsionen nach Anspruch 1 oder 2, **dadurch gekennzeichnet, dass** die Emulsion einen oder mehrere Öl-in-Wasser-Emulgatoren (O/W-Emulgatoren) in einer Gesamtkonzentration von 0,05 bis 5,0%, bezogen auf das Gesamtgewicht der Emulsion, beträgt.

4. O/W-Emulsionen nach einem der vorstehenden Ansprüche, **dadurch gekennzeichnet, dass** der Gehalt an einem oder mehreren anionischen Emulgatoren in der Emulsion von 0,001 bis 5,0 Gew.-% und bevorzugt zwischen 0,05 und 2 Gew.-%, besonders bevorzugt zwischen 0,1 und 1,0 Gew.-%, jeweils bezogen auf das Gesamtgewicht der Emulsion, beträgt.

5. O/W-Emulsionen nach einem der vorstehenden Ansprüche, **dadurch gekennzeichnet, dass** der Gehalt an einem oder mehreren nichtionischen Emulgatoren in der Emulsion von 0,001 bis 5,0 Gew.-% und bevorzugt zwischen 0,05 und 3 Gew.-%, besonders bevorzugt zwischen 0,1 und 2,0 Gew.-%, jeweils bezogen auf das Gesamtgewicht der Emulsion, beträgt.

## Claims

1. Cosmetic oil-in-water emulsions (O/W emulsions) comprising not only a water phase but also one or more oil phases dispersed therein, and
one or more anionic emulsifiers
and/or
one or more nonionic emulsifiers
and
one or more succinoglucans,
where polyglyceryl-3 methylglucose distearate (INCI: Polyglyceryl Methylglucose Distearate), potassium cetyl phosphate, sodium cetearyl sulfate, cetearyl glucoside, sodium stearyl glutamate, potassium cetyl phosphate, alkali metal salts of saturated fatty acids with a chain length of C14-C22 or one or more polyglyceryl fatty acid esters are used as O/W emulsifiers.

2. O/W emulsions according to Claim 1, **characterized in that** the content of succinoglucan in the emulsion is from 0.01% to 1.0% by weight, based on the total weight of the emulsion.

3. O/W emulsions according to Claim 1 or 2, **characterized in that** the emulsion is one or more oil-in-water emulsifiers (O/W emulsifiers) in a total concentration of 0.05% to 5.0%, based on the total weight of the emulsion.

4. O/W emulsions according to any of the preceding claims, **characterized in that** the content of one or more anionic emulsifiers in the emulsion is from 0.001% to 5.0% by weight and preferably between 0.05% and 2% by weight, particularly preferably between 0.1% and 1.0% by weight, in each case based on the total weight of the emulsion.

5. O/W emulsions according to any of the preceding claims, **characterized in that** the content of one or more nonionic emulsifiers in the emulsion is from 0.001% to 5.0% by weight and preferably between 0.05% and 3% by weight, particularly preferably between 0.1% and 2.0% by weight, in each case based on the total weight of the emulsion.

## Revendications

1. Émulsions cosmétiques huile-dans-eau (émulsions H/E) contenant, outre une phase aqueuse, une ou plusieurs phases huileuses dispersées dans celle-ci ainsi que
a) un ou plusieurs émulsifiants anioniques
et/ou
b) un ou plusieurs émulsifiants non ioniques
et
c) un ou plusieurs succinoglucanes,
où, en tant qu'émulsifiants H/E, sont utilisés du polyglyceryl-3 méthylglucose distéarate (INCl Polyglyceryl Methyl Glucose Distearate), du phosphate de cétyle de potassium, du sulfate de cétéaryle de sodium, du cétéarylglucoside, du glutamate de stéaryle de sodium, du phosphate de cétyle de potassium, des sels alcalins d'acides gras saturés de longueur de chaîne C14-C22 ou un ou plusieurs esters d'acides gras de polyglycéryle.

2. Émulsions H/E selon la revendication 1, **caractérisées en ce que** la teneur en succinoglucane dans l'émulsion est de 0,01 à 1,0 % en poids, par rapport au poids total de l'émulsion.

3. Émulsions H/E selon la revendication 1 ou 2, **caractérisées en ce que** l'émulsion est d'un ou plusieurs émulsifiants huile-dans-eau (émulsifiants H/E) à une concentration totale de 0,05 à 5,0 %, par rapport au poids total de l'émulsion.

4. Émulsions H/E selon l'une quelconque des revendications précédentes, **caractérisées en ce que** la teneur en un ou plusieurs émulsifiants anioniques dans l'émulsion est de 0,001 à 5,0 % en poids, et de préférence entre 0,05 et 2 % en poids, de manière particulièrement préférée entre 0,1 et 1,0 % en poids, chaque fois par rapport au poids total de l'émulsion.

5. Émulsions H/E selon l'une quelconque des revendications précédentes, **caractérisées en ce que** la teneur en un ou plusieurs émulsifiants non ioniques dans l'émulsion est de 0,001 à 5,0 % en poids, et de préférence entre 0,05 et 3 % en poids, de manière particulièrement préférée entre 0,1 et 2,0 % en poids, chaque fois par rapport au poids total de l'émulsion.
